# EUROPEAN PATENT SPECIFICATION

(11) **EP 1 186 301 B1**
(45) Date of publication and mention of the grant of the patent: **16.08.2006**
(21) Application number: 01122476.3
(22) Date of filing: 07.09.1994
(51) Int. Cl.: A61K 39/395

(54) **Monoclonal antibodies that recognize flk-2 receptors and the isolation of primitive hematopoietic stem cell populations**
Monoklonale Antikörper die Flk-2 Rezeptoren erkennen, und die Isolierung primitiver hämatopoietischer Stammzellpopulationen
Anticorps monoclonaux reconnaissant les récepteurs Flk-2 et isolement de populations de cellules souches primitives hématopoiétiques

(30) Priority: 08.09.1993 US 118468
(43) Date of publication of application: 13.03.2002
(62) Divisional of application: 94929168.6
(73) Proprietor: IMCLONE SYSTEMS, INC., New York, NY 10014 (US)
(72) Inventor: Goldstein, Neil I., Maplewood, NJ 07040 (US); Songsakphsan, Ratchaneem, Flushing, NY 11358 (US); Rose, Caroline, Jackson Heights, NY 11372 (US)
(74) Representative: Vogelsang-Wenke, Heike

(56) References cited:
- WO-A-94/01576

## Description

### FIELD OF THE INVENTION

The present invention relates to monoclonal antibodies that recognize the hematopoietic stem cell receptor known as flk-2 and to primitive hematopoietic stem cell populations that contain the flk-2 receptor.

### BACKGROUND OF THE INVENTION

The mammalian hematopoietic system comprises red and white blood cells. These cells are the mature cells that result from more primitive lineage-restricted progenitor cells. The cells of the hematopoietic system have been reviewed by Dexter and Spooncer in the Annual Review of Cell Biology 3, 423-441 (1987).

Red blood cells, or erythrocytes, result from primitive cells referred to by Dexter and Spooncer as erythroid burst-forming units (BFU-E). The immediate progeny of the erythroid burst-forming units are called erythroid colony-forming units (CFU-E).

The white blood cells contain the mature cells of the lymphoid and myeloid systems. The lymphoid cells include B lymphocytes and T lymphocytes. The B and T lymphocytes result from earlier progenitor cells referred to by Dexter and Spooncer as preT and preB cells.

The myeloid system comprises a number of cells including granulocytes, monocytes, macrophages, and megakaryocytes. The granulocytes are further divided into neutrophils, eosinophils, basophils and mast cells. The megakaryocytes shed portions of their cytoplasm to form platelets.

Each of the mature hematopoietic cells are specialized for specific functions. For example, erythrocytes are responsible for oxygen and carbon dioxide transport. T and B lymphocytes are responsible for cell- and antibody-mediated immune responses, respectively. Platelets are involved in blood clotting. Granulocytes and macrophages act generally as scavengers and accessory cells in the immune response against invading organisms and their by-products.

At the center of the hematopoietic system lie one or more totipotent hematopoietic stem cells, which undergo a series of differentiation steps leading to increasingly lineage-restricted progenitor cells. The more mature progenitor cells are restricted to producing one or two lineages. Some examples of lineage-restricted progenitor cells mentioned by Dexter and Spooncer include granulocyte/macrophage colony-forming cells (GM-CFC).

The hematopoietic system functions by means of a precisely controlled production of the various mature lineages. The totipotent stem cell is believed to possess the ability both to self renew and to differentiate into committed progenitors for all hematopoietic lineages. These most primitive of hematopoietic cells are both necessary and sufficient for the complete and permanent hematopoietic reconstitution of a radiation-ablated hematopoietic system in mammals. The ability of stem cells to reconstitute the entire hematopoietic system is the basis of bone marrow transplant therapy.

Human and mouse hematopoietic stem cells are characterized by the presence of certain antigens. For example, the presence of stem cell antigen 1 (Sca-1) and Thy-1 indicate the presence of murine hematopoietic stem cells. See Tsukamoto et al, European Patent Application 451,611. The presence of CD34, Thy-1, and Class II HLA (as well as its conserved DR epitope recognized by a monoclonal antibody called J1-43) all indicate the presence of human hematopoietic stem cells.

The antigens that are characteristic of murine hematopoietic stem cells, such as Sca-1 and Thy-1, and of human hematopoietic stem cells, such as Class II HLA, CD34+, and Thy-1, are not found exclusively on hematopoietic stem cells and early progenitor cells. Such antigens are also found on a significant number of more mature lineage committed cell populations.

Hematopoietic stem cells are further characterized by the absence of a number of other antigens that are found only on more mature cells. Some examples of human antigens found only on more mature hematopoietic cells include CD3, CD7, CD8,CD10, CD14, CD 15, CD19, CD20, CD33. Of these, CD10, CD19, and CD20 are associated with B cells; CD3, CD4, and CD8 are associated with T cells; and CD14, CD15, and CD33 are associated with myeloid cells.

The CD3, CD8, CD10, CD19, CD20, and CD33 antigens are collectively referred to as Lin. Accordingly, a highly purified human stem cell population is characterized by being CD34+ Lin-and CD34+ Lin- Thy-1+.

It is becoming increasingly apparent that the protein tyrosine kinases (pTKs) play an important role as cellular receptors for hematopoietic growth factors. Some receptor pTKs include c-kit and the receptors of colony stimulating factor 1 (CSF-1) and PDGF. These receptors, however, are found on mature hematopoietic cells as well as on primitive hematopoietic stem cells.

A receptor that is reported to be associated with primitive hematopoietic stem cells is the flk-2 receptor. The nucleotide sequence and amino acid sequence of the flk-2 receptor are given in International PCT Application US92/05401 of Ihor R. Lemischka as well as in Matthews et al., Cell 65, 1143-1152 (1991). This receptor defines a population of primitive hematopoietic stem cells (flk-2+).

Slightly different murine and intracellular human flk-2 nucleotide and amino acid sequences have been published by Rosnet et al., who referred to the receptor as flt-3; see Oncogene, 6, 1641-1650 (1991) and Genomics 9, 380-385 (1991), respectively. It is not known if the differences in the sequence reported in the Rosnet et al. articles and in the Lemischka patent application and Matthews et al. article are due to sequencing errors or to polymorphisms.

Various organs express the flk-2 receptor. Such organs include fetal liver, fetal spleen, fetal thymus, adult brain, and adult marrow. For example, flk-2 is expressed in individual multipotential CFU-Blast colonies capable of generating numerous multilineage colonies upon replanting.

The flk-2 receptor may be divided into easily found domains. The DNA sequence corresponding to the pTKs encode, starting at their 5' ends, a hydrophobic leader sequence followed by a hydrophilic extracellular domain. Immediately downstream from the extracellular receptor domain, is a hydrophobic transmembrane region. The transmembrane region is immediately followed by a basic catalytic domain.

The following table shows the nucleic acid and amino acid numbers that correspond to the signal peptide, the extracellular domain, the transmembrane region and the intracellular domain for murine flk-2 (mflk-2) and human flk-2 (hflk-2) as described in International PCT Application US92/05401.

**mflk-2**

| Signal Peptide | Extracellular | Transmembrane | Intracellular |
|---|---|---|---|
| aa no. -27 to -1 | 1 to 517 | 518 to 537 | 538 to 965 |
| aa code M T | N S | F C | H S |
| na no. 31-111 | 112-1662 | 1663-1722 | 1723-3006 |

**hflk-2**

| Signal Peptide | Extracellular | Transmembrane | Intracellular |
|---|---|---|---|
| aa no. -27 to -1 | 1 to 516 | 517 to 536 | 537 to 966 |
| aa code M N | Q F | Y C | H S |
| na no. 58-138 | 139-1689 | 1690-1746 | 1747-3036 |

### SUMMARY OF THE INVENTION

The object of the present invention is to provide antibodies that bind specifically to the flk-2 receptor and to cells that express the flk-2 receptor. Another object is to provide stem cell populations characterized by the presence of the flk-2 receptor. These and other objectives as will become apparent to those having ordinary skill in the art have been met by developing hybridoma cell lines that express rat anti-mouse flk-2 monoclonal antibody 2A13 (ATCC Accession Number HB 11444), rat anti-mouse flk-2 monoclonal antibody 23H (ATCC Accession Number HB 11443), rat anti-human flk-2 monoclonal antibody 19A (ATCC Accession Number HB 11442) and mouse anti-human flk-2 monoclonal antibody 6J11 (ATCC Accession Number HB 11445) and rat anti-human flk-2 monoclonal antibody 71E. Such antibodies permit the isolation of a hematopoietic stem cell population characterized by the presence of flk-2 receptors, and may also be used as agonists and antagonists of the flk-2 receptor.

The method of isolating a population of hematopoietic stem cells that expresses the flk-2 receptor from a mixture of hematopoietic cells comprises the steps of: (a) binding the cells that express the flk-2 receptor to an anti-flk-2 antibody; (b) separating the cells that express the flk-2 receptor bound to the anti-flk-2 antibody from the remainder of the mixture of hematopoietic cells; (c) unbinding the cells that express the flk-2 receptor from the anti-flk-2 antibody; and (d) isolating the cells that express the flk-2 receptor.

### DESCRIPTION OF THE FIGURES

Figure 1A. Titration of 23H7 by ELISA (Example 10).
Figure 1B. Titration of 2A13 by ELISA (Example 10).
Figure 2. FACS Analysis of saponin treated 3T3 cells and i3T3 cells with 2A 13 (Example 12).
Figure 3. Analysis of 2A13 and 23H7 binding to Flag-mflk-2 on the Biacore (Example 12).

### DETAILED DESCRIPTION OF THE INVENTION

The antibodies of the present invention are raised against, and bind specifically to, a protein tyrosine kinase called fetal liver kinase 2 (flk-2). For the purpose of this specification, flk-2 shall mean the tyrosine kinase receptor described by Lemischka in International PCT Application US92/05401 and in Cell 65, 1143-1152 (1991); as well as by Rosnet as flt-3 in Oncogene 6, 1641-1650 (1991) and in Genomics 9, 380-385 (1991). The antibodies are preferably raised against, and are specific for, the extra-cellular receptor domain of the flk-2 receptor.

The flk-2 receptor against which the antibodies are made may be bound to a cell. Alternatively, the flk-2 receptor may be free from the cell, preferably in soluble form.

### Functional Equivalents

The invention also includes functional equivalents of the antibodies described in this specification. Functional equivalents have binding characteristics comparable to those of the antibodies, and include, for example, single chain, chimerized and humanized antibodies. Chimerized antibodies preferably have constant regions derived substantially or exclusively from human antibody constant regions and variable regions derived substantially or exclusively from the sequence of the variable region from a mammal other than a human. Humanized antibodies preferably have constant regions and variable regions other than the complement determining regions (CDRs) derived substantially or exclusively from the corresponding human antibody regions and CDRs derived substantially or exclusively from a mammal other than a human. Suitable mammals other than a human include any mammal from which monoclonal antibodies may be made, such as a rabbit, rat, mouse, horse, goat, or primate.

Functional equivalents further include fragments of antibodies that have the same binding characteristics as, or that have binding characteristics comparable to, those of the whole antibody. Such fragments may contain one or both Fab fragments or the F(ab')₂ fragment. Preferably the antibody fragments contain all six complement determining regions of the whole antibody, although fragments containing fewer than all of such regions, such as three, four or five CDRs, may also be functional. Fragments may be prepared by methods described by Lamoyi et al in the Journal of Immunological Methods 56, 235-243 (1983) and by Parham in the Journal of Immunology 131, 2895-2902 (1983).

### Preparation of flk-2 receptors

In order to prepare the flk-2 receptors against which the antibodies are made, nucleic acid molecules that encode the flk-2 proteins of the invention, or portions thereof, especially the extracellular portions thereof, may be inserted into known vectors for expression using standard recombinant DNA techniques. Standard recombinant DNA techniques are described in Sambrook et al., "Molecular Cloning," Second Edition, Cold Spring Harbor Laboratory Press (1987) and by Ausubel et al. (Eds) "Current Protocols in Molecular Biology," Green Publishing Associates/ Wiley-Interscience, New York (1990). The vectors may be circular (i.e. plasmids) or non-circular. Standard vectors are available for cloning and expression in a host.

The host may be prokaryotic or eucaryotic. Prokaryotic hosts are preferably E. coli. Preferred eucaryotic hosts include yeast, insect and mammalian cells. Preferred mammalian cells include, for example, CHO, COS and human cells.

The DNA inserted into a host may encode the entire extracellular portion of the flk-2 receptor, or a soluble fragment of the flk-2 receptor. The extracellular portion of the flk-2 receptor encoded by the DNA is optionally attached at either, or both, the 5' end or the 3' end to additional amino acid sequences. The additional amino acid sequence may be attached to the flk-2 extracellular region in nature, such as those that represent the leader sequence, the transmembrane region and/or the intracellular region of flk-2. The additional amino acid sequences may also be sequences not attached to flk-2 in nature. Preferably, such additional amino acid sequences serves a particular purpose, such as to improve expression levels, solubility, or immunogencity. Some suitable additional amino acid sequences include, for example, the FLAG peptide (DYKDDDDKI) optionally attached at either end to the Fc portion of an immunoglobulin (Ig).

### Source of DNA Encoding flk-2 Receptors

In order to produce nucleic acid molecules encoding flk-2, a source of cells that express flk-2 is provided. Suitable fetal sources include liver, spleen, or thymus cells. Suitable adult sources include bone marrow, blood (peripheral or umbilical cord) or brain cells.

For example, suitable mouse fetal liver cells may be obtained at day 14 of gestation. Mouse fetal thymus cells may be obtained at day 14-18, preferably day 15, of gestation. Suitable fetal cells of other mammals are obtained at gestation times corresponding to those of the mouse gestation times.

### Isolation of Nucleic Acid Molecules Encoding flk-2 Receptors

Total RNA is prepared by standard procedures from flk-2 receptor-containing tissue or cells. The total RNA is used to direct cDNA synthesis. Standard methods for isolating RNA and synthesizing cDNA are provided in standard manuals of molecular biology such as, for example, in Sambrook et al., "Molecular Cloning," Second Edition, Cold Spring Harbor Laboratory Press (1987) and in Ausubel et al., (Eds), "Current Protocols in Molecular Biology," Greene Associates/Wiley Interscience, New York (1990).

The cDNA of the receptors may be amplified by known methods. For example, the cDNA may be used as a template for amplification by polymerase chain reaction (PCR); see Saiki et al., Science, 239, 487 (1988) or Mullis et al., U.S. patent 4,683,195. The sequences of the oligonucleotide primers for the PCR amplification are derived from the sequences of mouse and human flk-2 respectively. The oligonucleotides are synthesized by methods known in the art. Suitable methods include those described by Caruthers in Science 230, 281-285 (1985).

In order to isolate the entire protein-coding regions for the flk-2 receptors, the upstream PCR oligonucleotide primer is complementary to the sequence at the 5' end, preferably encompassing the ATG start codon and at least 5-10 nucleotides upstream of the start codon. The downstream PCR oligonucleotide primer is complementary to the sequence at the 3' end of the desired DNA sequence. The desired DNA sequence preferably encodes the entire extracellular portion of the flk-2 receptor, and optionally encodes all or part of the transmembrane region, and/or all or part of the intracellular region, including the stop codon. A mixture of upstream and downstream oligonucleotides are used in the PCR amplification. The conditions are optimized for each particular primer pair according to standard procedures. The PCR product may be analyzed by methods known in the art for cDNA having the correct size, corresponding to the sequence between the primers. Suitable methods include, for example, electrophoresis.

Alternatively, the coding region may be amplified in two or more overlapping fragments. The overlapping fragments are designed to include a restriction site permitting the assembly of the intact cDNA from the fragments.

The DNA encoding the flk-2 receptors may also be replicated in a wide variety of cloning vectors in a wide variety of host cells. The host cell may be prokaryotic or eukaryotic.

The vector into which the DNA is spliced may comprise segments of chromosomal, non-chromosomal and synthetic DNA sequences. Some suitable prokaryotic cloning vectors include plasmids from E. coli, such as colE1, pCR1, pBR322, pMB9, pUC, pKSM, and RP4. Prokaryotic vectors also include derivatives of phage DNA such as M13 and other filamentous single-stranded DNA phages.

### Expression and Isolation of Receptors

DNA encoding the receptors of the invention are inserted into a suitable expression vector and expressed in a suitable prokaryotic or eucaryotic host. Vectors for expressing proteins in bacteria, especially E.coli, are known. Such vectors include the PATH vectors described by Dieckmann and Tzagoloff in J. Biol. Chem. 260, 1513-1520 (1985). These vectors contain DNA sequences that encode anthranilate synthetase (TrpE) followed by a polylinker at the carboxy terminus. Other expression vector systems are based on beta-galactosidase (pEX); lambda P_{L}; maltose binding protein (pMAL); and glutathione S-transferase (pGST) -see Gene 67, 31 (1988) and Peptide Research 3, 167 (1990).

Vectors useful in yeast are available. A suitable example is the µ plasmid.

Suitable vectors for use in mammalian cells are also known. Such vectors include well-known derivatives of SV-40, adenovirus, retrovirus-derived DNA sequences and shuttle vectors derived from combination of functional mammalian vectors, such as those described above, and functional plasmids and phage DNA.

Further eukaryotic expression vectors are known in the art (e.g., P.J. Southern and P. Berg, J. Mol. Appl. Genet. 1, 327-341 (1982); S. Subramani et al, Mol. Cell. Biol. 1, 854-864 (1981); R.J. Kaufmann and P.A. Sharp, "Amplification And Expression Of Sequences Cotransfected with A Modular Dihydrofolate Reductase Complementary DNA Gene," J. Mol. Biol. 159, 601-621 (1982); R.J. Kaufmann and P.A. Sharp, Mol. Cell. Biol. 159, 601-664 (1982); S.I. Scahill et al, "Expression And Characterization Of The Product Of A Human Immune Interferon DNA Gene In Chinese Hamster Ovary Cells," Proc. Natl. Acad. Sci. USA 80, 4654-4659 (1983); G. Urlaub and L.A. Chasin, Proc. Natl. Acad. Sci. USA 77, 4216-4220, (1980).

The expression vectors useful in the present invention contain at least one expression control sequence that is operatively linked to the DNA sequence or fragment to be expressed. The control sequence is inserted in the vector in order to control and to regulate the expression of the cloned DNA sequence. Examples of useful expression control sequences are the lac system, the trp system, the tac system, the trc system, major operator and promoter regions of phage lambda, the control region of fd coat protein, the glycolytic promoters of yeast, e.g., the promoter for 3-phosphoglycerate kinase, the promoters of yeast acid phosphatase, e.g., Pho5, the promoters of the yeast alpha-mating factors, and promoters derived from polyoma, adenovirus, retrovirus, and simian virus, e.g., the early and late promoters or SV40, and other sequences known to control the expression of genes of prokaryotic or eukaryotic cells and their viruses or combinations thereof.

Vectors containing the receptor-encoding DNA and control signals are inserted into a host cell for expression of the receptor. Some useful expression host cells include well-known prokaryotic and eukaryotic cells. Some suitable prokaryotic hosts include, for example, E. coli, such as E. coli SG-936, E. coli HB 101, E. coli W3110, E. coli X1776, E. coli X2282 E. coli DHI, and E. coli MRCI, Pseudomonas, Bacillus, such as Bacillus subtilis, and Streptomyces. Suitable eukaryotic cells include yeast and other fungi, insect, animal cells, such as COS cells and CHO cells, human cells and plant cells in tissue culture.

Following expression in a host cell maintained in a suitable medium, the flk-2 receptors may be isolated from the medium, and purified by methods known in the art. If the flk-2 receptors are not secreted into the culture medium, the host cells are lysed prior to isolation and purification.

### Cells that Express flk-2 Receptors for Use as Antigens

Other sources of flk-2 receptors for preparing the antibodies of the invention are flk-2 receptors bound to the surface of cells that express the flk-2 receptor. The cells to which the flk-2 receptors are bound may be a cell that naturally expresses the receptor, such as a hematopoietic stem cell. Alternatively, the cell to which the full length or truncated receptor is bound may be a cell into which the DNA encoding the receptor has been transfected, such as 3T3 cells.

Preferred sources of mammalian stem cells that express flk-2 receptors for use as antigens to prepare anti-flk-2 antibodies include bone marrow and adult peripheral or umbilical cord blood. Stem cells may be isolated from bone marrow or blood in accordance with methods known in the art.

### Preparation of Antibodies

The antibodies may be polyclonal or monoclonal. The antibodies may be further modified by methods known in the art, as discussed above.

The antibodies are preferably monoclonal. Monoclonal antibodies may be produced by methods known in the art. These methods include the immunological method described by Kohler and Milstein in Nature 256, 495-497 (1975) and Campbell in "Monoclonal Antibody Technology, The Production and Characterization of Rodent and Human Hybridomas" in Burdon et al., Eds, Laboratory Techniques in Biochemistry and Molecular Biology, Volume 13, Elsevier Science Publishers, Amsterdam (1985); as well as by the recombinant DNA method described by Huse et al in Science 246, 1275-1281 (1989).

### Isolation of Population of Stem Cells Expressing the FLK-2 Receptor

A source of hematopoietic stem cells is provided. The source will normally be a mixture of hematopoietic cells that comprises the desired population of stem cells expressing the flk-2 receptor as well as more mature cells that do not express the flk-2 receptor. Suitable fetal sources of hematopoietic stem cells include liver, spleen, or thymus. Suitable adult sources of hematopoietic stem cells include bone marrow, blood (peripheral or umbilical cord) or brain cells. The preferred source is human adult bone marrow.

Suitable hematopoietic stem cells may be harvested from a suitable mammalian donor by methods known in the art. For example, bone marrow may be obtained from a human patient undergoing an autologous bone marrow transplant. Alternatively, bone marrow may be obtained from a suitable bone marrow donor participating in an allogenic transplant.

The source of stem cells, such as blood or bone marrow cells, are exposed to an antibody that binds to the flk-2 receptor, such as an antibody of the present invention, preferably the 19A monoclonal antibody or the 6J11 monoclonal antibody.

The cells that express the flk-2 receptor, i.e. the flk-2+ population of cells, are bound to the anti-flk-2 antibody. The cells bound to the antibodies are optionally separated from the unbound cells by methods known in the art, and then are isolated free from the antibody. For example, the cells may be separated from the antibodies by treatment with a protease, such as chymotrypsin.

In one embodiment, an anti-flk-2 antibody of the invention is attached to a solid support. Some suitable solid supports include nitrocellulose, agarose beads, polystyrene beads, hollow fiber membranes, and plastic petrie dishes. For example, the antibody can be covalently linked to Pharmacia Sepharose 6MB macrobeads. The exact conditions and duration of incubation for the solid phase-linked antibodies with the marrow cell suspension will depend upon several factors specific to the system employed, as is well known in the art.

Cells that are bound to the antibody are removed from the cell suspension by physically separating the solid support from the cell suspension. For example, the unbound cells may be eluted or washed away with physiologic buffer after allowing sufficient time for the solid support to bind the stem cells.

The bound cells are separated from the solid phase by any appropriate method, depending mainly upon the nature of the solid phase and the antibody. For example, bound cells can be eluted from a plastic petrie dish by vigorous agitation. Alternatively, bound cells can be eluted by enzymatically "nicking" or digesting an enzyme-sensitive "spacer" sequence between the solid phase and the antibody. Suitable spacer sequences bound to agarose beads are commercially available from, for example, Pharmacia.

The eluted, enriched fraction of cells may then be washed with a buffer by centrifugation and preserved in a viable state at low temperatures for later use according to conventional technology. The cells may also be used immediately, for example by being infused intravenously into a transplant recipient.

In a particularly preferred variation of the method described above, blood is withdrawn directly from the circulatory system of a donor. The blood is percolated continuously through a column containing the solid phase-linked monoclonal antibody to remove stem cells. The stem cell-depleted blood is returned immediately to the donor's circulatory system using by methods known in the art, such as hemapheresis. The blood is processed in this way until a sufficient number of stem cells binds to the column. This method allows rare peripheral blood stem cells to be harvested from a very large volume of blood, sparing the donor the expense and pain of harvesting bone marrow and the associated risks of anesthesia, analgesia, blood transfusion, and infection.

Stem cell suspensions produced by the methods described above consist essentially of populations of cells that express the flk-2 receptor (flk-2+). Such cell suspensions are substantially free of mature lymphoid and myeloid cells.

Other methods for isolating purified populations of flk-2+ antibodies are also known Such methods include magnetic separation with antibody-coated magnetic beads, affinity chromatography, cytotoxic agents, such as complement, conjugated to the antibody, and "panning" with an antibody attached to a solid matrix.

### General Fluorescence Activated Cell Sorting (FACS) Protocol

In a preferred embodiment, a labelled antibody of the invention is bound to flk-2+ cells, and the labelled cells are separated by a mechanical cell sorter that detects the presence of the label. The preferred mechanical cell sorter is a florescence activated cell sorter (FACS). FACS machines are commercially available. Generally, the following FACS protocol is suitable for this procedure:

A Coulter Epics Eliter sorter is sterilized by running 70% ethanol through the systems. The lines are flushed with sterile distilled water.

Cells are incubated with a primary antibody, such as 19A or 6J11, diluted in Hank's balanced salt solution supplemented with 1% bovine serum albumin (HB) for 60 minutes on ice. The cells are washed with HB and incubated with a secondary antibody labelled with fluorescein isothiocyanate (FTTC) for 30 minutes on ice. The secondary label binds to the primary antibody. For example, in the case of 19A, a suitable secondary antibody is goat anti-rat-FTTC. In the case of 6J11, a suitable secondary antibody is goat anti-mouse-FITC. The sorting parameters, such as baseline fluorescence, are determined with an irrelevant primary antibody. The final cell concentration is usually set at one million cells per ml.

While the cells are being labelled, a sort matrix is determined using fluorescent beads as a means of aligning the instrument.

Once the appropriate parameters are determined, the cells are sorted and collected in sterile tubes containing medium supplemented with fetal bovine serum and antibiotics (usually penicillin, streptomycin and/or gentamycin. After sorting, the cells are re-analyzed on the FACS to determine the purity of the sort.

### Flow Cytometry

Another method for isolating such flk-2+ populations of cells is flow cytometry; see, for example, Loken et al., European Patent Application 317,156. The cells obtained may be assayed, and in general used, by engraftment into radiation-ablated mammals by methods known in the art for reconstituting the hematopoietic system of the mammal as described below; see, for example, Jordan et al., Cell 61, 953-963 (1990).

### Pre-purified Stem Cell Population

Before crude hematopoietic cell populations are treated with antibodies of the invention, the cells are preferably pre-purified by methods known in the art. For example, human hematopoietic stem cells may be purified by means of the anti-My-10 monoclonal antibody described by Civin in U.S. patent 5,130,144. The hybridoma cell line that expresses the anti-My monoclonal antibody is available from the American Type Culture Collection, 12301 Parklawn Drive, Rockville, Maryland 20852, USA. The anti-My-10 monoclonal antibody binds to the CD34 antigen. Cell populations consisting essentially of CD34 expressing cells are referred to as CD34+ population of stem cells. Some additional sources of antibodies capable of selecting CD34+ cells include AMAC, Westbrook, Maine; Coulter, Hialea, Florida; and Becton Dickinson, Mountain View, California. CD34+ cells may also be isolated by means of comparable antibodies, which may be produced by methods known in the art, such as those described by Civin in U.S. Patent 5,130,144.

The CD34+ population of human hematopoietic stem cells described above is preferably further purified by means of the Thy-1 antibody and/or Lin panel of antibodies by methods known in the art; see Tsukamoto et al. U.S. patent 5,061,620. The preferred cell population used to prepare the flk-2+ stem cell population with the antibodies of the invention are, therefore preferably CD34+, more preferably CD34+ Lin-, and more preferably CD34+ Lin- Thy-1+. Such cell populations are preferably further purified so that they are Class II HLA+ by means antibodies against Class II HLA (including its conserved DR epitope recognized by a monoclonal antibody called J1-43).

### UTILITY

The flk-2+ cell populations of the invention can be used to restore the cells of the erythroid, myeloid, or lymphoid systems in a mammal that has lost the ability to produce such cells as a result, for example, of radiation treatment. Suitable mammals include, for example, mice, rats, rabbits, and humans. Preferably, the flk-2+ cell populations are used to restore two of the systems, and more preferably are used to restore all three of the systems.

Accordingly, the flk-2+ stem cell populations of the invention can be used in therapeutic methods such as stem cell transplantation as well as others that are readily apparent to those of ordinary skill in the art. For example, such cell populations can be administered intravenously to a patient requiring a bone marrow transplant, such as, for example, radiation-ablated mammals, in an amount sufficient to reconstitute the patient's hematopoietic and/or immune system, or a portion of the patient's hematopoietic and immune system.

The number of cells in the flk-2+ stem cell population sufficient to reconstitute the patient's hematopoietic and/or immune system can be readily determined by those skilled in the art. This number will depend upon the exact condition being treated by the therapy as well as relevant particulars of the mammal, such as age, size, health, etc. In general, an amount of flk-2+ stem cells containing approximately the same number of stem cells found in one-half to one liter of aspirated marrow is adequate.

In addition to the therapeutic uses described above, the flk-2+ cell population may be used as a starting material to produce other valuable materials. For example, the cells may be used as a source of the flk-2 receptors or DNA encoding the receptors, or as an antigen for making monoclonal and polyclonal antibodies that recognize and bind to the flk-2 receptor or other antigens on the surface of flk-2+ cells.

The antibodies of the present invention may be used to isolate and purify soluble flk-2 receptors as well as flk-2+ hematopoietic stem cells, as described above. In addition, the antibodies may also be used diagnostically to monitor the level of the flk-2+ stem cells or of antibodies against the flk-2+ receptor in biological samples. Some examples of biological samples include bodily fluids, such as bone marrow or blood. Such diagnostic use involves, for example, labelling the antibodies and conducting standard immunoassays, such as radioimmunoassays, as is well know in the art.

The antibodies may also be used to detect the presence of flk-2 containing cells, such as tumor cells, in vitro or to image such cells in vivo. Suitable tumor cells include leukemia cells, such as, for example, acute myelogenous leukemias and acute lymphocytic leukemia. The antibodies are labelled and detected or imaged by methods known in the art.

The antibodies may also be used in vitro or in vivo to mimic , i.e. to act as agonists of, flk-2 receptor growth factors due to the ability of the antibodies to bind to the flk-2 receptor. Such agonists are desirable to cause the growth of cells that express the flk-2 receptor, such as hematopoietic stem cells. Such agonists are also desirable to prevent the growth of cells that express or overexpress the flk-2 receptor, such as tumor cells, especially tumor cells of the hematopoietic system, i.e., the leukemia cells mentioned above, by causing terminal differentiation. For example, some of the antibodies of the invention are able to simulate flk-2 receptor growth factors by stimulating flk-2 receptor-containing cells to proliferate and/or to differentiate.

Alternatively, the antibodies of the invention may be used as antagonists of flk-2 receptor growth factors. Such antagonists are desirable to prevent the growth of cells that express or overexpress the flk-2 receptor, such as tumor cells, especially tumor cells of the hematopoietic system, i.e., leukemic cells. For example, the binding of antibodies to the flk-2 receptor may reduce or prevent the proliferation and/or differentiation of flk-2+ cells by preventing the binding of the flk-2 receptor growth factor to the cells. The binding of antibodies to the flk-2 receptor on flk-2+ cells may also be toxic to the cells by, for example, antibody depenedent cellular cytotoxicity (ADCC).
If the antibodies are not toxic, or are insufficiently toxic, by themselves, the antibodies may be made toxic, or may be made more toxic, by the administration of the antibodies to a mammal in combination with one or more cytotoxins. The antibodies and cytotoxins may be administered to a mammal, such as a mammal described above, separately, or the antibodies may be attached to the cytotoxins by, for example, conjugation, as is known in the art. Some suitable toxins include, for example, reicin and gelonin.

The flk-2+ stem cell populations of the invention can be used in therapeutic compositions, optionally in the presence of pharmaceutically acceptable excipients, as will be readily apparent to those of ordinary skill in the art. Such therapeutic compositions can be administered by methods known in the art, for example, intravenously, to a mammal requiring an agonist or antagonist of flk-2 growth factors. Examples of mammals requiring an agonist or antagonist of flk-2 growth factors include tumor-bearing mammals. The antibodies of the invention are administered in an amount sufficient for the antibodies to act as agonists or antagonists of flk-2 growth factors by, for example, inhibiting the growth of tumors.

### Examples

### Example 1. Flag-flk-2 expression plasmids

A synthetic DNA fragment (fragment 1) is synthesized using complementary oligonucleotides BP1 and BP2 (see below and SEQ. ID. NOS. 1 and 2). Fragment 1 encodes the following features in the 5' to 3' order: Sal I restriction site, 22 base pair (bp) 5' untranslated region containing an eukaryotic ribosome binding site, an ATG initiation codon, preprotrypsinogen signal sequence, coding region for the FLAG peptide (DYKDDDDKI) and Bgl II restriction site.

A cDNA fragment (Fragment 2) encoding Asn 27 to Ser 544 of murine flk-2 is obtained by polymerase chain reaction (PCR) using primers designed to introduce an in-frame Bgl II site at the 5' end (oligonucleotide BP5, see below and SEQ. ID. NO. 3) and a termination codon followed by a Not I site at the 3' end (oligonucleotide BP10, see below and SEQ. ID. NO. 4). The template for the PCR reaction is full length flk-2 cDNA (Matthews et al., Cell 65, 1143-1152 (1991)). Fragment 2 is extensively digested with Bgl II and Not I restriction enzymes prior to ligation.

To assemble the complete Flag-flk-2 gene, Fragments 1 and 2 are ligated in a tripar ligation into Sal I and Not I digested plasmid pSPORT (Gibco/BRL, Grand Island, NY) to give the plasmid pFlag-flk-2.

Preferably, the Flag-flk-2 protein is attached at either end to the Fc portion of an immunoglobulin (Ig). The Ig is preferably attached to the flk-2 portion of the Flag-flk-2 protein. To assemble the construct pFlag-flk-2-Ig, the sequences coding for the CH¹ doma of human immunoglobulin G (IgG¹) are placed downstream of the flk-2 coding region in tl plasmid pFlag-flk-2 in accordance with the method described by Zettlemeissl et al., DNA a Cell Biology 9: 347-352 (1990).

The sequences of oligonucleotides used to construct the Flag-flk-2 gene are given below:
Oligonucleotide BP1: 
5'-AATTCGTCGACTTTCTGTCACCATGAGTGCACTTCTGATCCTAGCCCTTGTG
GGAGCTGCTGTTGCTGACTACAAAGATGATGATGACAAGATCTA-3'
Oligonucleotide BP2: 
5'-AGCTTAGATCTTGTCATCATCATCTTTGTAGTCAGCAACAGCAGCTCCCACA
AGGGCTAGGATCAGAAGTGCACTCATGGTGACAGAAAGTCGACG-3'
Oligonucleotide BP5:
   5'-TGAGAAGATCTCAAACCAAGACCTGCCTGT-3'
Oligonucleotide BP10:
   5'-CCAATGGCGGCCGCTCAGGAGATGTTGTCTTGGA-3'
(See SEQ. ID. NOS. 1-4, respectively)

### Example 2. Expression of the Flag-flk-2 construct

For transient expression of the Flag-flk-2 construct, the Sal 1 to Not I fragment from pFlag-flk-2 is subcloned into the plasmid pSVSPORT (Gibco/BRL) to give the plasmid pSVFlag-flk-2. For expression of the Flag-flk-2 protein, pSVFlag-flk-2 is transfected into COS monkey cells using the DEAE-dextran method.

For stable expression in eukaryotic cells, the Sal I-Not I fragment of pFlag-flk-2 is cloned into the EcoRV and Not I sites of the plasmid pcDNA I/Neo (Invitrogen Co., San Diego, CA). The Sal I 3' recessed terminus of pFlag-flk-2 is filled with the Klenow fragment of DNA polymerase I and a mixture of deoxyribonucleotides to make the site compatible with the EcoRV site of the vector. The resulting construct is introduced into cultured mammalian cells using either the Lipofectin (Gibco/BRL) or the calcium phosphate methods.

For expression in insect cells, the Sail to Hind III (from pSPORT polylinker) fragment of pFlag-flk-2 is subcloned into the BamH 1-Hind III sites of the baculovirus transfer vector pBlueBac III (Invitrogen). The vector Bam HI site and the insert Sal I site are blunted with Klenow (see above). Production of the recombinant virus and infection of the Sf9 insect cells is performed as per manufacturers directions (Invitrogen).

Expression of the Flag-flk-2 protein is detected by Western blotting of SDS-PAGE separated conditioned media (mammalian cells) or cell lysates (insect cells) with the anti-Flag monoclonal antibody (mAb) M 1 (International Biotechnology, Inc. (IBI), New Haven, CT).

### Example 3. Purification of Recombinant Flag-flk-2

Affinity purification of the Flag-flk-2 protein from COS conditioned media or insect cell lysates was performed as per manufacturers specifications using the anti-Flag monoclonal antibody M 1 immobilized on agarose (International Biotechnology, Inc., New Haven, CT.)

Baculovirus-derived Flag-flk-2 was purified from harvested SF-9 cells 48-72 hours after infection. Cells were collected by centrifugation at 3500 x g for 10 minutes at 4C and washed with ice-cold PBS. The cell pellet was resuspended and homogenized on ice in 50mM Tris-HCI, pH 7.4, 500mM NaCI, 300mM sucrose, 2mM CaCl₂O, .05% Tween 80, 10 g/ml leupeptin, 1 g/ml pepstatin, and 1mM PMSF. The cell lysate was centrifuged at 100000 x g for 60 minutes at 4C and the supernatant fraction collected. The clarified cell lysate was loaded onto a 3 ml anti-Flag M 1-agarose column (IBI,) pre-equilibrated in Buffer A (0.1 M Tris-HCl, pH7.4, 0.5M NaCl, 2 mM CaCl₂, 0.05% Tween 80). After passing the sample 5 times over the M 1 resin, the column was washed with 60 ml of Buffer A and eluted with 30 ml of Buffer B (0.1 M Tris-HCI, pH 7.4, 0.5 M NaCI, 10 mM EDTA, 0.05% Tween 80). Fractions that contained flk-2 receptor were pooled, dialyzed into PBS, and frozen at -70C. Protein was quantitated by the BCA method (Pierce,) using bovine serum albumin as the standard.

### Example 4A. Preparation of the Anti-mouse flk-2 monoclonal antibodies 2A 13 (IgG2a) and 23H (IgG1)

Monoclonal antibodies were prepared using two protocols. In the first, female Lewis rats (Charles River) were immunized with Flag-flk-2 (obtained from COS cell-conditioned media in accordance with Example 3) bound to the anti-Flag monoclonal antibody, M1, coupled to agarose. On day 0, rats received 100 ul of the resin-protein complex in incomplete Freund's adjuvant (IFA) by the subcutaneous route. Rats received subsequent injections of the complex on day 26 (plus Ribi adjuvant) and day 62 (without adjuvant). At various times, the animals were bled to determine antibody titer. The rats received two additional subcutaneous immunizations on days 91 and 105 of purified baculovirus-derived Flag-flk-2 (10 and 40g/injection, respectively; both with Ribi adjuvant). Four days after the final inoculation, one rat was sacrificed and the spleen asceptically removed for isolation of primed B cells.

In the second protocol, male Lewis rats (Charles River) were immunized with purified baculovirus-derived Flag-flk-2 by the intraperitoneal route on day 0 (25g in complete Freund's adjuvant); day 7 (25g in Ribi adjuvant). Bleeds were obtained at various times to determine anti-flk-2 titer. Four days after the final injection, one animal was sacrificed and the spleen removed for subsequent fusion.

Splenocytes obtained by either protocol were fused to the murine myeloma cell line NS 1 using polyethylene glycol (PEG 3350; Baker) as described previously; see Goldstein et al., Anticancer Research 10, 491 (1990). The cells were resuspended in HT (hypoxanthine/thymidine; Sigma) medium consisting of Dulbecco's modified Eagle's medium (DMEM) supplemented with 20% heat inactivated fetal bovine serum (Hyclone), L-glutamine (2mM), HT supplement (Flow Labs), and antibiotics and plated at a concentration of 1.6 x 10⁵ cells per well in 96 well microtiter plates. On day one, medium was replaced with the above medium containing HAT (instead of HT). After 10-14 days, macroscopic colonies were visible and ready for the initial screen.

### Example 4B. Preparation of the Anti-Human flk-2 Monoclonal Antibodies 19A (IgG2B) and 6J11 (IgG1)

Rat monoclonal antibody 19A was generated by hyperimmunizing female Lewis rats (Charles River Laboratories) with a purified preparation of soluble human Flag-flk-2 expressed in Cos-7 cells (Examples 2 and 3). Murine monoclonal antibody 6J11 was generated by hyperimmunizing female Balb/c mice (Charles River Laboratories) as above. All injections were given intraperitoneally using the following schedule:

For 19A, rats previously immunized with murine Flag-flk-2 were injected on day 0 (10 ug in Ribi adjuvant), day 7 (10 ug without Ribi) and day 14 (15 ug plus 20 ug of mutein IL-6). For 6J11, mice previously immunized with a human flk-2 protein-expressing leukemic cell line ML-1, were injected on day 0 (10 ug plus Ribi adjuvant), day 7 (10 ug plus 20 ug of IL-6) and day 14 (10 ug plus 20 ug IL-6). (Comparable results are obtained with other human flk-2 protein-expressing leukemic cell lines, such as HL-60 (ATCC) and KG-1A (ATCC)). Prior to fusion, animals were bled and the sera screened by ELISA for binding activity to purified human flk-2.

Three to four days following the final injection, animals were sacrificed and the spleens asceptically removed. The splenocytes were obtained by mechanical disruption of the spleens, washed in sterile serum-free medium (usually Dulbecco's modified Eagle's medium) and mixed with NS1, a murine myeloma cell line. Fusion was accomplished by the stepwise addition of one ml of polythylene glycol (50% in serum free medium; PEG 3600; JT Baker) over 2 minutes followed by dilution with 10 ml of serum free medium. Cells were centrifuged (200 x g for 5 minutes), resuspended at 1-5 x 10 5 cells per ml in HT medium (Dulbecco's modified Eagle's medium supplemented with 10% fetal bovine serum, 2 mM L-glutamine, antibiotics and 1xHT media supplement and 100 ul aliquoted into each well of several 96 well tissue culture plates. After 24 hours at 37C, 100 ul of HAT medium was added (HT medium plus 1xHAT (hypoxanthine/aminopterin/thymidine); Sigma) for selection purposes.

Colonies were seen at 10 to 20 days in culture. Hybridoma supernatants initially screened by ELISA. In this assay, 25 ng of purified human flk-2 protein, in carbonate buffer, pH 9.6, was coated (overnight at 4C) on 96 well microtiter plates. Plates were blocked with Hank's balanced salt solution containing 1% BSA for 60 minutes at 37C. After extensive washing, a horseradish peroxidase-labelled secondary antibody (goat anti-mouse Ig for 6J11 or goat anti-rat Ig for 19A) was added for 60 minutes at 37C. Positive binding was determined using the chromogen TMB and plates were read in an ELISA reader.

### Example 5. ELISA Protocol

Purified baculovirus-derived Flag-flk-2 was coated onto 96 well microtiter plates (50ng/well) using 50 mM sodium carbonate buffer, pH 9.6. After an overnight incubation at 4C, plates were washed two times in distilled water and blocked with 10% new born calf serum (Hyclone) in PBS (NB10) for 30 minutes at 37C. The conditioned supernatants from the fusions were added to the aspirated wells and incubated for two hours at 37C. The wells were washed three times in distilled water and probed with a goat anti-rat secondary antibody conjugated to horseradish peroxidase (HRP; Tago). After a one hour incubation at 37C, the wells were extensively washed with distilled water and the chromogen substrate (TMV; Kirkegaard and Perry, Gaithersburg, Maryland) added. The reaction was stopped with 1N sulfuric acid and the plates read at 450nm in an automated ELISA reader.

After cloning (see below), monoclonal antibodies were isotyped using a capture ELISA. Plates were coated with goat anti-rat Ig overnight at 4C. After blocking, supernatants were added for two hours at 37C, washed and probed with isotype specific, using TMB as the chromogen as described above.

### Example 6. Cloning Protocols

Based on the results of the ELISA's a number of hybridoma colonies were picked and expanded onto 24 well microtiter plates. Supernatants were re-screened for binding to purified baculo-derived Flag-flk-2 and cloned three times by limiting dilution (1 cell per well). Clones were assayed for flk-2 binding by ELISA.

### Example 7. Western Blot Protocols

The following cell lines were grown to confluency in 75cm² flasks: NIH3T3 and i3T3 (whole mflk-2 transfected 3T3 cells). Media was apirated and the cells washed twice with PBS. One ml of lysis buffer (consisting of 10mM Tris HCl, pH 8.0, 0.2% Triton X-100, 150mM NaCI, 2mM EDTA, 1mM PMSF, and 5g/ml of Aprotinin) was added to the flasks which were kept on ice until the cells were seen to lyse. Adherent material was removed by scrapping with a rubber policeman and kept on ice for an additional hour. Cell debris was removed by centrifugation at 10000 rpm in a bench top microfuge maintained at 4C. The protein concentration of the lysate was determined using the Biorad assay prior to separation by SDS PAGE.

Five microgram aliquots in SDS sample buffer (2% SDS, 5% 2-mercaptoethanol, 10% glycerol, 60 mM Tris-HCl, 0.01% bromophenol blue, pH 6.8) were run on 10-20% gradient gels (Enprotech, Natick MA) as described (Laemli Nature 227, 680 (1970). Following the electrophoresis, the bands were transferred to Immobilon-P membrane. Blots were stored at 4C for a maximum of two days before performing Western Blot analysis; see Goldstein et al., Anticancer Research 10, 491 (1990). The membrane was blocked with NB-1 containing 0.1 % Triton X-100 for one hour at room temperature and incubated with hybridoma conditioned medium with added 0.1% Triton X-100 for one hour with rocking. The membrane was washed three times with PBS containing 0.1% Triton X-100 and incubated for one hour with goat anti-rat HRP conjugated secondary antibody (1:1000; Tago, Burlingame, California). The membrane was washed three times with PBS containing 0.1% Triton X-100 and incubated for one hour with goat anti-rat HRP conjugated secondary antibody (1:1000; Tago). The membrane was washed three times and the bands developed with TMB containing membrane enhancer solution (Kirkegaard and Perry). After the appearance of bands, the membrane was washed in cold tap water.

### Example 8. Metabolic Labelling and Immunoprecipitation Analysis

NIH-3T3 and i3T3 Flk-2 transfectants (i3T3) were seeded into 100 mm dishes and grown to near confluency. The cells were starved in methionine and cysteine-free medium (Gibco, Grand Island, NY) for 30 minutes followed by incubation in methionine and cysteine-free medium containing 0.2 mCi/ml Tran-³⁵S-label (ICN, Amersham) for 48 hours. Labelled cells were washed three times with PBS and lysed in IP Buffer (0.1% NP-40, 50 mM Tris-HCl, 150 mM NaCl, 1 mM EDTA, 0.2% deoxycholate, 0.1% SDS, 1 mM PMSF, pH 7.4). Aliquots of 500 ul of cell lysate were immunoprecipitated with 5 ug of purified monoclonal antibody 23H. Immune complexes were absorbed with goat anti-rat Ig (Tago,) coupled to Protein A Sepharose. The immune complexes were washed several times in IP Buffer and solubilized by boiling for 2 minutes in electrophoresis sample buffer. Proteins were resolved on 8% polyacrylamide/SDS gels as described. After electrophoresis, gels were fixed in 40% methanol/7% acetic acid for one hour, washed in Enlightening (NEN, Boston MA) for one hour, dried and autoradiographed at -70°C.

### Example 9. FACS Analysis

Cells for analysis are removed from T flasks using non-enzymatic dissociation buffer (Sigma), counted, and aliquoted into 12x75mm glass tubes at a final concentration of approximately one million cells per tube. All steps are performed on ice to prevent internalization of the receptor antibody complexes. Cells are pelleted at 1000 rpm and resuspended in 100 ul of primary antibody solution:100 ul of conditioned supernatant or 5ug per tube of purified antibody in Hank's balanced salt solution containing 1% BSA (HBSS-B). After a one hour incubation, cells are resuspended in 1 ml of HBSS-B, pelleted, and the supernatant discarded. Goat anti-rat IgG conjugated to FITC (5ug/tube; Tago) is added to each tube for an additional 30-60 minute incubation protected from light. Cells are washed three times with HBSS-B, resuspended in 1 ml of HBSS-B and analyzed at once. Otherwise, cells are fixed with 1 % neutral buffered formalin and analyzed within 48 hours. In some experiments, saponin was used to expose FLK-2 epitopes. For these studies, 0.1% saponin was included in all incubations and washes.

The binding of the anti-flk-2 monoclonal antibodies to murine fetal liver derived stem cells was screened as follows. Pregnant Balb/c mice (untimed pregnancies, about fourteen days; Charles River Labs) were sacrificed and the fetuses removed. Liver tissue was asceptically collected and disrupted on ice. The resulting cell suspension was treated with 0.17M ammonium chloride to remove red blood cells, washed twice with HBSS-B, and resuspended in cold HBSS-B at a final concentration of approximately ten million cells per ml. One hundred milliter aliquots were added to glass tubes and incubated for one hour on ice with various antibodies including 2A13, 23H, AA4.1, DC101 (a rat IgG1 monoclonal antibody directed to the flk-1 receptor), and irrelevant rat IgG (Tago). After washing the cells, goat anti-rat IGG-FITC was added and incubated for 30 minutes on ice. The cells were extensively washed and analyzed as described above.

FITC-labelled cells were analyzed with a Coulter Elite cell sorter using the Multigraph program. For every cell line, an isotype matched irrelevant rat monoclonal antibody was run to determine baseline flourescence.

### Example 10. Specificity of Murine Monoclonal Antibodies 2A13 and 23H

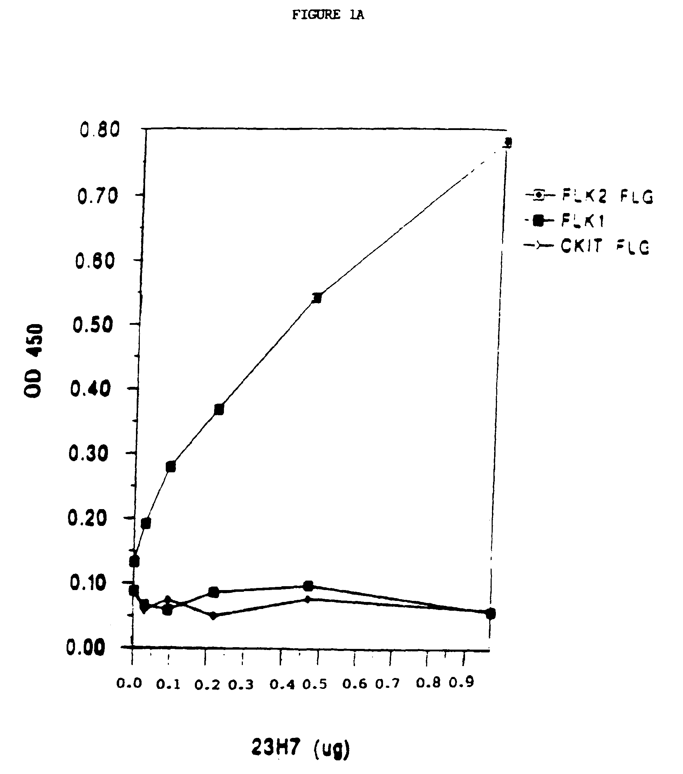

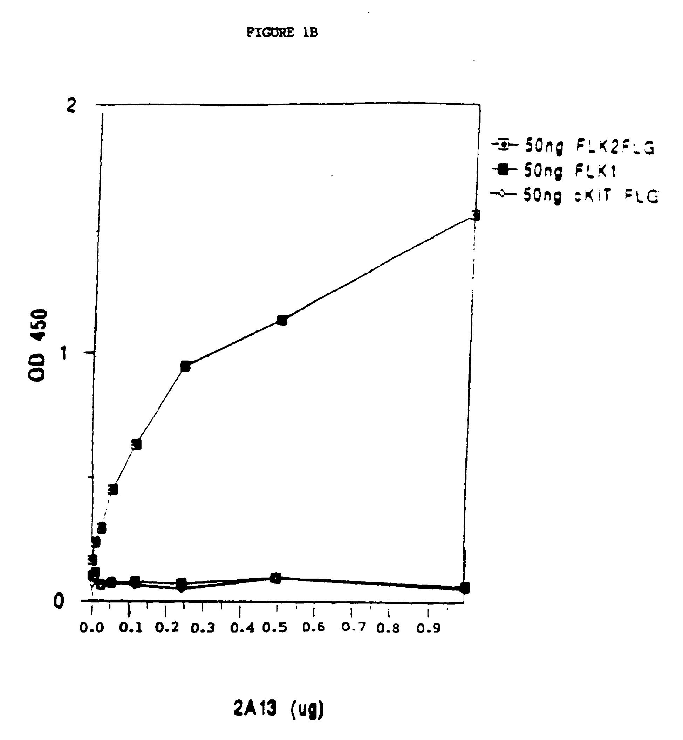

Initially, the monoclonal antibodies were screened against mouse and human flk-2 and other related protein tyrosine kinases including c-kit and flk-1 using an ELISA. As can be seen in Figure 1, both antibodies bind to Flag-flk-2 but not to either c-kit or flk-1 (US92/05401). Neither monoclonal antibody appeared to bind to the human receptor.

### Example 11. Immunoprecipitation of murine flk-2 antibodies with monoclonal antibodies 2A13 and 23H

Monoclonal antibodies 2A13 and 23H are able to immunoprecipitate radiolabelled soluble and cellular murine flk-2. Each antibody immunoprecipitates soluble Flag-flk-2 as a single protein as evidenced by the presence of a single band by SDS PAGE of approximately 95kD, which is the estimated molecular weight for this construct. 23H is also able to immunoprecipitate whole flk-2 from the transfected cell line i3T3 as evidenced by the presence of two bands by SDS PAGE of approximate molecular weights of 135kD and 155kD, which are the estimated molecular weights of cellular flk-2 with different glycosylation patterns.

### Example 12. Binding Studies with Monoclonal Antibodies 2A13 and 23H

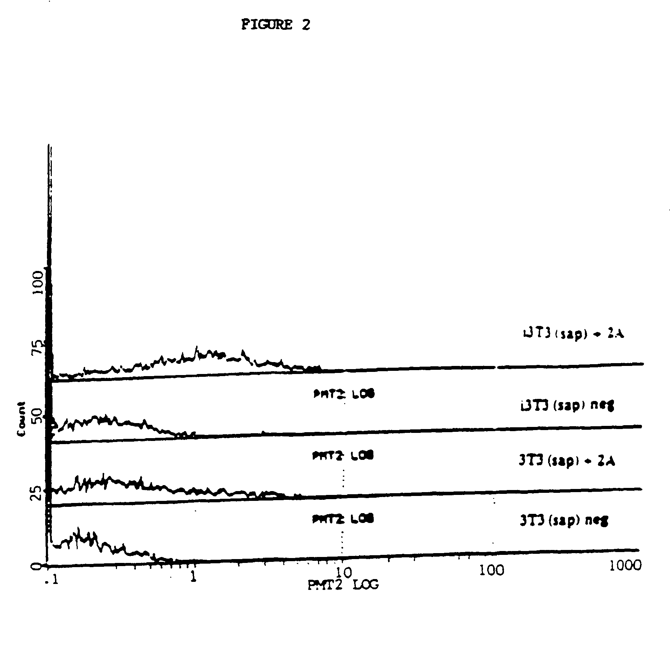

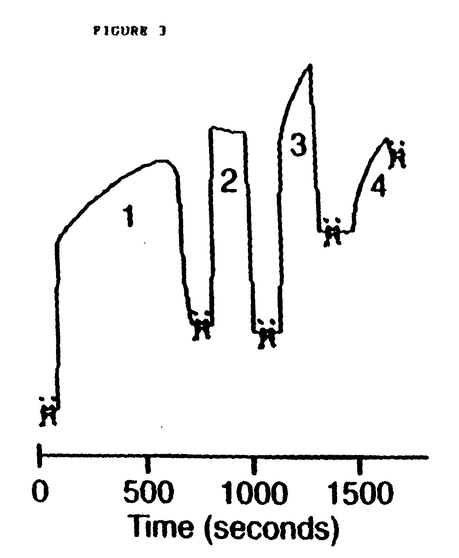

2A13 and 23H were analyzed on the FACS for their ability to bind to cells expressing FLK-2. Figure 2 shows the results of these studies. Both 2A13 and 23H bound to i3T3 but not untransfected 3T3 cells. However, only 23H bound to intact viable cells whereas 2A13 required prior saponin treatment (0.1%) of the target cells for binding. This appears to indicate that the epitope recognized by 2A13 is inaccessible to the monoclonal antibody as opposed to the availability of the 23H determinant. That these two monoclonal antibodies recognize two distinct epitopes was confirmed by studies using an instrument that measures real-time biospecific interactions based on surface plasmon resonance (BIAcore, Pharmacia Biosensor AB). As can be seen in Figure 3, the binding of 2A13 does not interfere with subsequent binding of 23H.

### Example 13. Cells that Express Full Length flk-2 Receptors (Y-stk)

Y-stk cells are prepared by co-transfecting DNA coding for the human flk-2 receptor and neo resistance into NIH 3T3 cells. Transfected cells are selected in medium containing 1 mg/ml of G418. Viable colonies, appearing after two to three weeks, are isolated, expanded, and screened for the presence of huma flk-2 using Western blot analysis. For these studies, transfected colonies are lysed, resuspended in SDS sample buffer, and separated by SDS PAGE. Proteins are blotted onto nitrocellulose paper and probed with IM140, a rabbit polyclonal antibody that was prepared to the C-terminal amino acid sequence of flk-2. One colony, designated Y-stk, was found to express human flk-2 and was propagated for subsequent studies.

### Example 14. Rat Monoclonal Antibody 71E (IgG2B)

Rats were hyperimmunized with Y-stk on days zero, 15, 30, 44, 51, and 61. Serum was collected at various times during the immunization schedule to check for the presence of a humoral response to the receptor. Four days after the final (day 61) injection, two animals were sacrificed and the spleens isolated and processed for hybridization.

Hybridomas were initially screened by ELISA (see Example 15 below) using unfixed Y-stk cells. Subsequent screening assays utilized cell lysates (see Example 16 below) and FACS analysis as described above for 19A4B et al. A hybridoma called 71E was found to be positive in these assays and was cloned by limiting dilution. Hybridoma 71E expresses monoclonal antibody 71E.

Monoclonal antibody 71E binds to human flk-2 by ELISA; binds to human flk-2 expressing cells by FACS; immunoprecipitates a diffuse band in the flk-2 region that corresponds to the bands immunoprecipitated by a rabbit polyclonal antibody specific to the C-terminal region of flk-2 (Im 140); blocks the phosphorylation of the human flk-2 receptor by its natural ligand (see Example 17 below); and binds to human flk-2 expressing leukemic cells KG la (ATCC) but not flk-2-negative cells when screened by FACS analysis.

### Example 15. ELISA Protocol.

96 well microtiter plates are coated with 1 ug/well of IM 140. Plates are washed and blocked with Hanks balanced salt solution supplemented with 1% BSA Five to 10 micrograms of Y-stk or control 3T3 lysate (see Example 16 below) are then added each well. After a two hour incubation at 37C, plates are washed and binding probed with goat anti-rat IgG conjugated to horse radish peroxidase followed by the addition of the chromogen TMB. Plates are read at 450 nm.

### Example 16. Preparation of Cell Lysates.

Y-stk or control 3T3 cells are grown to confluency in large petri dishes. Cells are scraped off the plates with a rubber policeman, collected by centrifugation, and resuspended in lysis buffer (0.02 M Tris-HCL, pH 7.2, 50 mM NaCl, 10 mM EDTA, 1% Triton X-100, 10% glycerol, 100 ug/ml Aprotinin, 500 ug/ml leupeptin, 400 ug/ml Pefabloc). Lysates are checked for human flk-2 by Western blot analysis and stored at -70C.

### Example 17. Phosphorylation Assay.

Y-stk or control 3T3 cells are grown to 90% confluency in DME-supplementd with 10% calf serum and then serum starved in DME-0.5% CS for 24 hours prior to experimentation. For neutralization assays, cells were stimulated with various concentrations of the flk-2 ligand under serum free conditions (DME -0.1% BSA) in the presence and absence of monoclonal antibody 71E or irrelevant rat antibody for 15 minutes at room temperature. The antibodies were assayed at 10 and 20 ug/ml. To evaluate the effects of antibody on the flk-2 ligand-induced activation of its cognate receptor, antibody was either added simultaneously (competitive inhibition) or prebound to cells for 15 minutes at room temperature prior to the addition of flk-2 ligand. SeeLyman et al., Cell, 75, 1157-1167 (1993); Blood 83, 2795-2801 (1994); Oncogene 8, 815-822 (1993). Cells incubated in serum free medium in the absence and presence of antibody served as controls for receptor autophosphorylation in the absence of ligand and the presence of antibody, respectively.

Following stimulation, monolayers were washed with ice cold PBS containing 1mM sodium orthovanadate. Cells were then lysed in lysis buffer [(20 mM Tris-HCl, pH 7.4, 1% Triton X-100, 137mM NaCl, 10% glycerol, 10 mM EDTA, 2mM sodium orthovanadate, 100 mM NaF, 100mM sodium pyrophosphate, 5mM Pefabloc (Boehringer Mannheim Biochemicals, Indianapolis IN), 100 ug aprotinin and 100 ug/ml leupeptin] and centrifuged at 14000 x g for 10 minutes. Protein was immunoprecipitated from lysates of transfected cells using IM140 coupled to Protein A Sepharose beads. The beads were then washed once with 0.2% Triton X-100, 10 mM Tris-HCl pH8.0, 150 mM NaCl, 2mM EDTA (Buffer A), twice with Buffer A containing 500 mM NaCl and twice with Tris-HCl, pH 8.0. Drained beads were mixed with 30 ul in 2x SDS loading buffer and subjected to SDS PAGE in 4-12% gradient gels. After electrophoresis, proteins were blotted to nitrocellulose filters for analysis. To detect phosphorylated receptor, blots were probed with a monoclonal antibody to phosphotyrosine (UBI, Lake Placid, NY) and developed by an enhanced chemiluminescence system (ECL, Amershaw).

### SUPPLEMENTAL ENABLEMENT

The invention as claimed is enabled in accordance with the above specification and readily available references and starting materials. Nevertheless, Applicants deposited on August 24, 1993 with the American Type Culture Collection, Rockville, Md., USA (ATCC) the hybridoma cell lines that produce the monoclonal antibodies listed below:
Rat anti-mouse flk-2 monoclonal antibody 2A13 (ATCC Accession Number HB 11444);
Rat anti-mouse flk-2 monoclonal antibody 23H (ATCC Accession Number HB 11443);
Rat anti-human flk-2 monoclonal antibody 19A (ATCC Accession Number HB 11442); and
Mouse anti-human flk-2 monoclonal antibody 6J11 (ATCC Accession Number HB 11445).
Rat anti-human flk-2 monoclonal antibody 71E (ATCC Accession Number HB).

These deposits were made under the provisions of the Budapest Treaty on the International Recognition of the Deposit of Microorganisms for the Purposes of Patent Procedure and the regulations thereunder (Budapest Treaty). This assures maintenance of a viable culture for 30 years from date of deposit. The organisms will be made available by ATCC under the terms of the Budapest Treaty, and subject to an agreement between Applicants and ATCC which assures unrestricted availability upon issuance of the pertinent U.S. patent. Availability of the deposited strains is not to be construed as a license to practice the invention in contravention of the rights granted under the authority of any government in accordance with its patent laws.

### SEQUENCE LISTING

(1) GENERAL, INFORMATION:
   (i) APPLICANT: Goldstein, Neil I.
      Songsakphisarn, Ratchanee
      Rose, Caroline
   (ii) TITLE OF INVENTION: MONOCLONAL ANTIBODIES THAT RECOGNIZE FLK-2 RECEPTORS AND THE ISOLATION OF PRIMATIVE HEMATOPOIETIC STEM CELL POPULATIONS
   (iii) NUMBER OF SEQUENCES: 4
   (iv) CORRESPONDENCE ADDRESS:
      (A) ADDRESSEE: ImClone Systems Incorporated
      (B) STREET: 180 Varick Street
      (C) CITY: New York
      (D) STATE: New York
      (E) COUNTRY: U.S.A.
      (F) ZIP: 10014
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25
   (vi) CURRENT APPLICATION DATA:
      (A) APPLICATION NUMBER: US
      (B) FILING DATE: 08-SEP-1993
      (C) CLASSIFICATION:
   (viii) ATTORNEY/AGENT INFORMATION:
      (A) NAME: Feit, Irving N.
      (B) REGISTRATION NUMBER: 28,601
      (C) REFERENCE/DOCKET NUMBER: GOL-2
   (ix) TELECOMMUNICATION INFORMATION:
      (A) TELEPHONE: 212-645-1405
      (B) TELEFAX: 212-645-2054
(2) INFORMATION FOR SEQ ID NO:1:
   (i) SEQUENCE CHARACTERISTICS:
      (A) LENGTH: 96 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (xi) SEQUENCE DESCRIPTION: SEQ ID NO: 1:
      AATTCGTCGA CTTTCTGTCA CCATGAGTGC ACTTCTGATC CTAGCCCTTG TGGGAGCTGC 60
      TGTTGCTGAC TACAAAGATG ATGATGACAA GATCTA 96
(2) INFORMATION FOR SEQ ID NO:2:
   (i) SEQUENCE CHARACTERISTICS:
      (A) LENGTH: 96 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (xi) SEQUENCE DESCRIPTION: SEQ ID NO:2:
      AGCTTAGATC TTGTCATCAT CATCTTTGTA GTCAGCAACA GCAGCTCCCA CAGAGGCTAG 60
      GATCAGAAGT GCACTCATGG TGACAGAAAG TCGACG 96
(2) INFORMATION FOR SEQ ID NO:3:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (xi) SEQUENCE DESCRIPTION: SEQ ID NO:3:
      TGAGAAGATC TCAAACCAAG ACCTGCCTGT 30
(2) INFORMATION FOR SEQ ID NO:4:
   (i) SEQUENCE CHARACTERISTICS:
      (A) LENGTH: 34 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (xi) SEQUENCE DESCRIPTION: SEQ ID NO:4:
      CCAATGGCGG CCGCTCAGGA GATGTTGTCT TGGA 34

## Claims

1. Use of an antagonist antibody directed against flk-2 for the preparation of a medicament for the treatment of leukaemia.

## Patentansprüche

1. Verwendung eines gegen flk-2 gerichteten antagonistischen Antikörpers zur Herstellung eines Medikaments für die Behandlung von Leukämie.

## Revendications

1. Utilisation d'un anticorps antagoniste dirigé contre le flk-2 pour la préparation d'un médicament pour le traitement de la leucémie.
